# EUROPEAN PATENT APPLICATION

(11) **EP 1 500 369 A1**
(43) Date of publication of application: **26.01.2005**
(21) Application number: 03723202.2
(22) Date of filing: 25.04.2003
(51) Int. Cl.: A61B 5/145

(54) **BIOLOGICAL INFORMATION DETECTING CONTACT**

(30) Priority: 01.05.2002 JP 2002129622; 19.06.2002 JP 2002178448; 28.10.2002 JP 2002313234
(71) Applicant: Matsushita Electric Industrial Co., Ltd., Kadoma-shi, Osaka 571-8501 (JP)
(72) Inventor: OOSHIMA, Kiyoko, Shijonawate-shi, Osaka 575-0024 (JP); UCHIDA, Shinji, Neyagawa-shi, Osaka 572-0807 (JP); SHIOI, Masahiko, Katano-shi, Osaka 576-0021 (JP)
(74) Representative: Grünecker, Kinkeldey, Stockmair & Schwanhäusser Anwaltssozietät
(86) International application number: PCT/JP2003/005297
(87) International publication number: WO 2003/092499

(57) **Abstract**

With a conventional technique, it is difficult to ensure adhesion of a prism and a portion-under-measurement and a measurement position reproducibility, and measurement data easily vary.

An information detecting contact shoe 5 contacting on a portion-under-measurement irradiates light, and at the time of detection of transmitted light, reflected light, scattered light or transmitted and reflected light from the portion-under-measurement and non-invasive measurement of a particular component contained in the portion-under-measurement, exposing means 8a is utilized which exposes a labial mucosa of a living body's lip portion which is the portion-under-measurement. Further, a prism 5 is attached to the exposing means 8a.

## Description

### FIELD OF THE INVENTION

The present invention relates to a vital information measuring device or the like which takes non-invasive measurement of vital information, such as a blood sugar value and cholesterol of a living body, utilizing transmitted light, reflected light, scattered light or transmitted and reflected light.

### BACKGROUND ART

According to public data included in the "survey on the actualities of diabetes" which the Ministry of Welfare announced as for 1997, a population of those who were strongly suspected to be diabetics was 6.9 million and a population of these people combined with people with an undeniable possibility of suffering diabetes reached 13.7 million.

Diabetes often accompanies no subjective symptoms, and among those who are strongly suspected to suffer diabetes, people who are currently treated for diabetes account for 45%. The present situation is that although diabetes is a serious disease which often leads to complications and kills people, due to lack of subjective symptoms except for a high blood sugar value in the initial stage, the seriousness of this disease fails to be recognized very well. People therefore could loose a chance of getting cured by revising their living practices, which is said to be the biggest problem regarding diabetes.

While severe diabetes patients personally own blood-collecting type blood sugar measuring devices which can be easily operated and rely on a method that they collect their own blood themselves and measure their blood sugar values, those people whose subjective symptoms are only to such an extent that they could have a possibility of diabetes and healthy people with no possibility of diabetes are less interested in blood sugar values, and even when they are interested in blood sugar values, they would not personally own blood-collecting type blood sugar measuring devices and habitually take measurement.

Noting this, over the recent years, such devices have been proposed with which it is possible to take non-invasive measurement of the blood sugar value of a subject who is under measurement. For instance, a method requiring to measure while holding an ATR (attenuated total reflection) prism with mouth or between fingers has been proposed as a method which, using an ATR prism for example, makes incident light repeat attenuated total reflection at a boundary of a reflection surface of the prism and a living body, and which then analyzes light emitted toward outside the prism (e.g., Japanese Patent Application Laid-Open Gazette No. H9-113439).

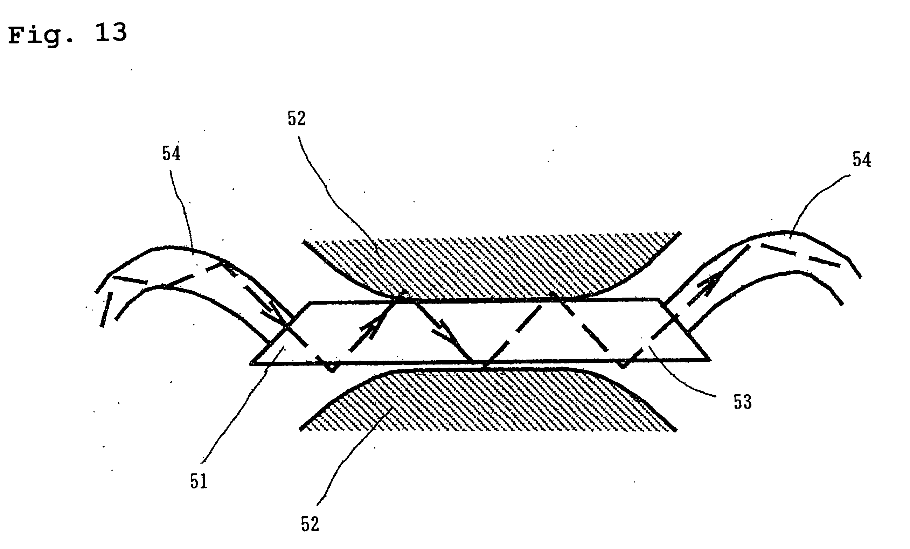

This method is an application of an effusing wave (so-called evanescent light) to quantitative analysis. As shown in Fig. 13, light 53 propagating through an optical fiber 54 and moving forward in an ATR prism 51 slightly intrudes lips 52 and then gets reflected by the lips 52. Since the light 53 thus intrudes the lips 52 and gets exposed to an influence of each component included in a humor which is present in the lips 52, measurement of the amount of the reflected light permits to detect changes of the reflectance, the absorption and the like of the humor and hence to obtain information regarding each component included in the humor. The entire disclosure of Japanese Patent Application Laid-Open Gazette No. H9-113439 is incorporated herein by quotation (reference) is its entirety.

However, the conventional measuring device described above has the following problem.

Now, calculating the effusing depth by a known formula as for an example that the wavelength of light is 10 microns, ZnSe crystals (refractive index: approximately 2.0) is used as the ATR prism 51, the angle of incidence is 45 degrees and a surrounding medium is water (refractive index: approximately 1.24), the effusing depth is 4.7 microns. Further, while the effusing depth changes when a surrounding refractive index changes, these changes if any are at most a few microns.'

In other words, while it is seen that information regarding a surface of a living body and the state of a neighboring section is obtained, in this case however it is hard to obtain information regarding a deep part of the living body at or beyond a few microns, and further, the presence of a disturbance layer such as saliva in particular between the living body and a subject-under-measurement changes the depth at which light fails to reach or intrudes the living body and accordingly makes a signal instable.

Hence, when one holds the ATR prism 51 between the lips as in the conventional example described above, the degree of close adhesion changes depending upon how strongly the one closes the lips. Thus, the adhesion of the prism surface and the lips is largely influenced by saliva instead of staying stable.

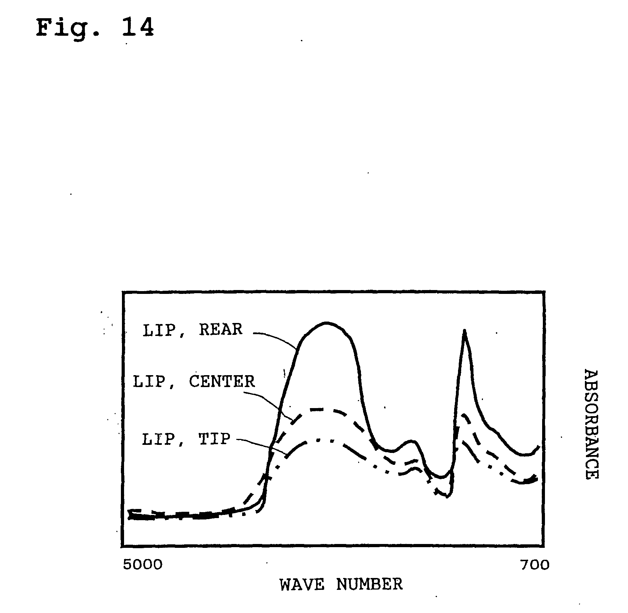

In addition, it is necessary to ensure that measurement conditions, particularly, the relative positional relationship between the prism and a portion-under-measurement of the living body such as a lip, are constant. Fig. 14 shows an example of spectra measured on a lip, in which the spectral profiles are considerably different from each other among different portions-under-measurement, such as the tip of the lip, an approximately central section of the lip and a rear section of the lip, and thus contains too great variations to be analyzed as spectral profiles containing the same information.

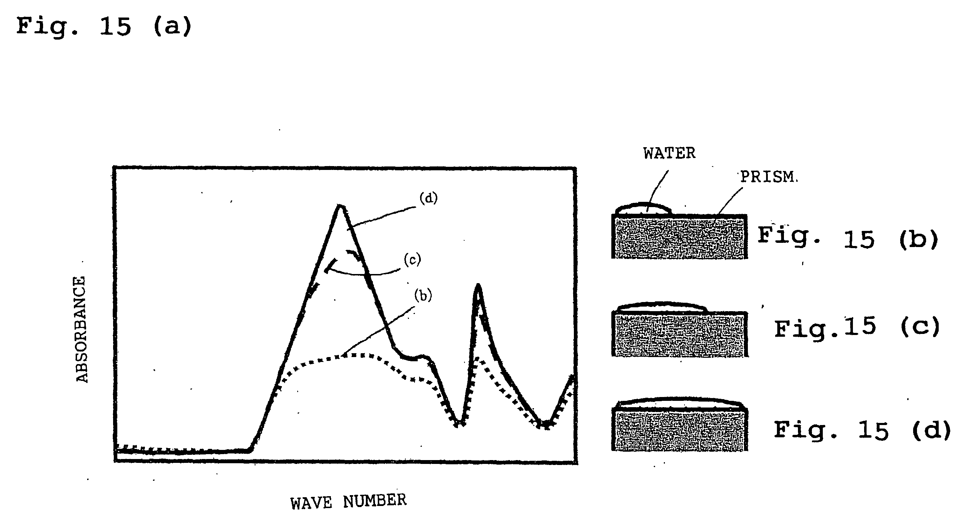

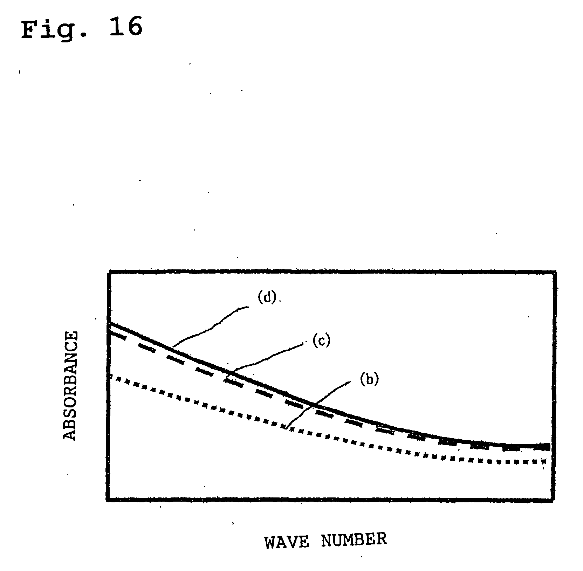

Fig. 15(a) shows an example of spectra measured on water, and Fig. 16 partially expands the spectra, and between a situation where there is water and air locally at a sensing portion of the ATR prism 51 as shown in Figs. 15 (b) and (c) and a situation where there is water entirely at the sensing portion of the ATR prism 51 as shown in Fig. 15 (d) for example, spectral profiles are different. That is, the layer of intervening air also serves as a cause of variations.

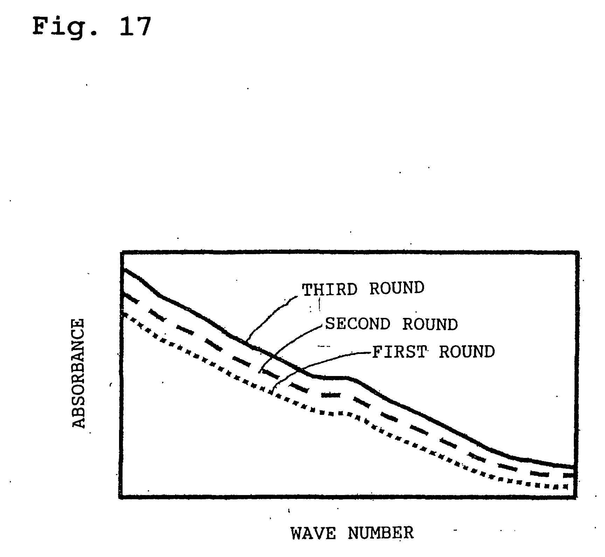

Fig. 17 shows a partially expanded example of spectra measured on the lips 52, for which using a clip-shaped pressing member incorporating an elastic member such as a spring for close adhesion with the ATR prism, measurement was taken successively three times with the lips 52 and the ATR prism 51 closely adhering to each other. The spectra have changed from the first round of measurement to the third round of measurement, which means that the thickness of a saliva layer has changed. The change of the saliva layer represents a phenomenon attributed to gradual changes of the pressing force for the purpose of pressing the soft lips 52 merely by means of elastic force, and as such, serves as a cause of variations.

Hence, when one presses the ATR prism 51 against the lips 52 while holding the ATR prism 51 between the lips 52 as in the conventional example described above, the degree of close adhesion changes depending upon how strongly one closes the lips 52, the close adhesion of the surface of the ATR prism 51 and the lips 52 is not stable, it is difficult to control the thickness of a saliva layer, and due to an influence exerted by the layer of intervening air or the like, measurement data tend to vary.

Further, even after wiping off of the saliva layer, a labial mucosa has a minutely uneven surface which gives rise to an air layer when adhesion is insufficient.

In addition, propagation of light in the optical fiber 54 further affects an instable signal accompanying a large propagation loss, which easily tends to vary measurement data.

### SUMMARY OF THE INVENTION

In light of these point, the present invention aims at providing a method and a device of measuring vital information, with which it is easy to achieve close adhesion between and the reproducibility of a position on a prism and a portion-under-measurement and it is possible to measure at a high accuracy.

A first invention of the present invention is a vital information detecting contact device, comprising:
first contacting means having an contacting surface which contacts on a portion-under-measurement of a subject-under-measurement; and
fixing means which fixes such that said contacting surface will not move on said portion-under-measurement,
wherein said contacting surface comprises an outgoing area, from which light leaves to said portion-under-measurement, and an incident area upon which all or some of transmitted light, reflected light, scattered light or transmitted and reflected light based on light thus leaving said outgoing area from said portion-under-measurement impinges upon.

A second invention of the present invention is the vital information detecting contact device of the first invention of the present invention, comprising deforming means which deforms at least a part of said subject-under-measurement such that said portion-under-measurement will be able to contact on said contacting surface.

A third invention of the present invention is the vital information detecting contact device of the second invention of the present invention, wherein said subj ect-under-measurement is a lip portion of a living body, said portion-under-measurement is a labial mucosa of said lip portion, and
said deforming means deforms said lip portion such that said labial mucosa will be exposed to outside.

A fourth invention of the present invention is the vital information detecting contact device of the third invention of the present invention, wherein said deforming means comprises press-open means which is held inside the mouth of said living body and heads toward a frenulum labii inferioris from gum outside teeth.

A fifth invention of the present invention is the vital information detecting contact device of the second invention of the present invention, comprising a fitting part which meshes with upper and lower teeth inside the mouth of said living body,
wherein said first contacting means is disposed outside the row of teeth, and as said fitting part meshes with said upper and lower teeth, said contacting surface closely adheres to said labial mucosa.

A sixth invention of the present invention is the vital information detecting contact device of the third invention of the present invention, wherein said deforming means comprises firm holding means which pinches said lip portion at one place at least.

A seventh invention of the present invention is the vital information detecting contact device of the sixth invention of the present invention, wherein said firm holding means is structured so as to revolve about a sulcus mentolabialis or a portion below the sulcus mentolabialis.

An eighth invention of the present invention is the vital information detecting contact device of the seventh invention of the present invention, wherein said fixing means comprises holding means which holds said first contacting means, moves when said firm holding means revolves in association with the revolution of said firm holding means, and makes said contacting surface contact on said lip portion.

A ninth invention of the present invention is the vital information detecting contact device of the first invention of the present invention, wherein said subject-under-measurement is a living body, and
said fixing means realizes said fixing when supported by at least one of the row of teeth, the jaw and the head of said living body.

A tenth invention of the present invention is the vital information detecting contact device of the first invention of the present invention, further comprising second contacting means which is located facing said first contacting means beyond said portion-under-measurement, and contacts on a different portion from said portion-under-measurement of said subject-under-measurement, and
said fixing means fixes so that said first contacting means and said second contacting means will be maintained at a constant distance with each other.

An eleventh invention of the present invention is the vital information detecting contact device of the tenth invention of the present invention, wherein said distance fixing means is capable of setting said constant distance to a plurality of distances which are different from each other.

A twelfth invention of the present invention is the vital information detecting contact device of the tenth invention of the present invention, wherein the surface area size of said contacting surface is smaller than the surface area size of a portion on which said second contacting means contacts.

A thirteenth invention of the present invention is the vital information detecting contact device of the tenth invention of the present invention, wherein said second contacting means has a substantially convex shape.

A fourteenth invention of the present invention is the vital information detecting contact device of the thirteenth invention of the present invention, wherein said convex shape has a round part.

A fifteenth invention of the present invention is the vital information detecting contact device of the tenth invention of the present invention, comprising pressure measurement means which measures the pressure upon said portion-under-measurement applied by said first contacting means and/or said second contacting means.

A sixteenth invention of the present invention is the vital information detecting contact device of the fifteenth invention of the present invention, wherein said pressure measurement means changes said constant distance based on said measured pressure.

A seventeenth invention of the present invention is a vital information measuring device, comprising:
the vital information detecting contact device of any one of the first through the sixteenth inventions;
a light source which sends light to said outgoing area of said contacting surface;
light receiving means which receives light from said incident area of said contacting surface; and
calculating means which calculates a light receipt result of said light receiving means and acquires information regarding said portion-under-measurement.

An eighteenth invention of the present invention is the vital information measuring device of the seventeenth invention of the present invention, further comprising confirmation means which confirms a place deformed by said deforming means.

A nineteenth invention of the present invention is the vital information measuring device of the eighteenth invention of the present invention, wherein said confirmation means is contact confirmation by means of a contact sensor.

A twentieth invention of the present invention is the vital information measuring device of the eighteenth invention of the present invention, wherein said contact confirmation utilizes an end portion of said press-open means.

A twenty-first invention is directed to the vital information measuring device according to the seventeenth invention, comprising:
light splitting means which splits light from said light source;
light guiding means which guides light from said light splitting means to said outgoing area; and
a holding member which holds as one unit said light source, said light splitting means, said light guiding means, said light receiving means and said vital information detecting contact device.

A twenty-second invention is directed to the vital information measuring device according to the twenty-first invention, wherein said light guiding means is means which uses a mirror.

A twenty-third invention is directed to the vital information measuring device according to the twenty-second invention, wherein a position restricting member is attached to said holding member, and using this position restricting member, the position of said information detecting contact device is restricted.

A twenty-fourth invention is directed to a vital information measuring method, comprising the steps of:
making an contacting surface, which comprises an outgoing area, from which light leaves toward a portion-under-measurement of a living body, and an incident area, upon which all or some of transmitted light, reflected light, scattered light or transmitted and reflected light based on light coming from said outgoing area from said portion-under-measurement, contact on said portion-under-measurement; and
fixing such that said contacting surface will not move on said portion-under-measurement,
wherein based on light obtained from said incident area, a particular component contained in said portion-under-measurement is measured in a non-invasive manner.

A twenty-fifth invention is directed to the vital information measuring method according to the twenty-fourth invention, further comprising a step of deforming said subject-under-measurement at least partially so that said portion-under-measurement will contact on said contacting surface.

A twenty-sixth invention is directed to a vital information measuring method which requires to irradiate light from an information detecting contact shoe which contacts on a portion-under-measurement, to detect transmitted light, reflected light, scattered light or transmitted and reflected light from this portion-under-measurement, and to measure a particular component contained in the portion-under-measurement in a non-invasive manner,
wherein after a labial mucosa of a living body's lip portion which is a portion-under-measurement is exposed, said information detecting contact shoe is made closely adhere to this labial mucosa.

A twenty-seventh invention is directed to the vital information measuring method according to the twenty-sixth invention, wherein said labial mucosa is exposed, as a contacting member contacting on a lip front surface is moved along a jaw direction.

A method invention relevant to the present invention is directed to a vital information measuring method which requires to irradiate light from an information detecting contact shoe which contacts on a portion-under-measurement, to detect transmitted light, reflected light, scattered light or transmitted and reflected light from this portion-under-measurement, and to measure a particular component contained in the portion-under-measurement in a non-invasive manner,
wherein at the time that light from a light source is split, thus split light is guided to said information detecting contact shoe, transmitted light, reflected light, scattered light or transmitted and reflected light from said portion-under-measurement is detected, thus detected signal is computed, and vital information is obtained,
the position of said information detecting contact shoe at least is adjusted.

### BRIEF DESCRIPTION OF THE DRAWINGS

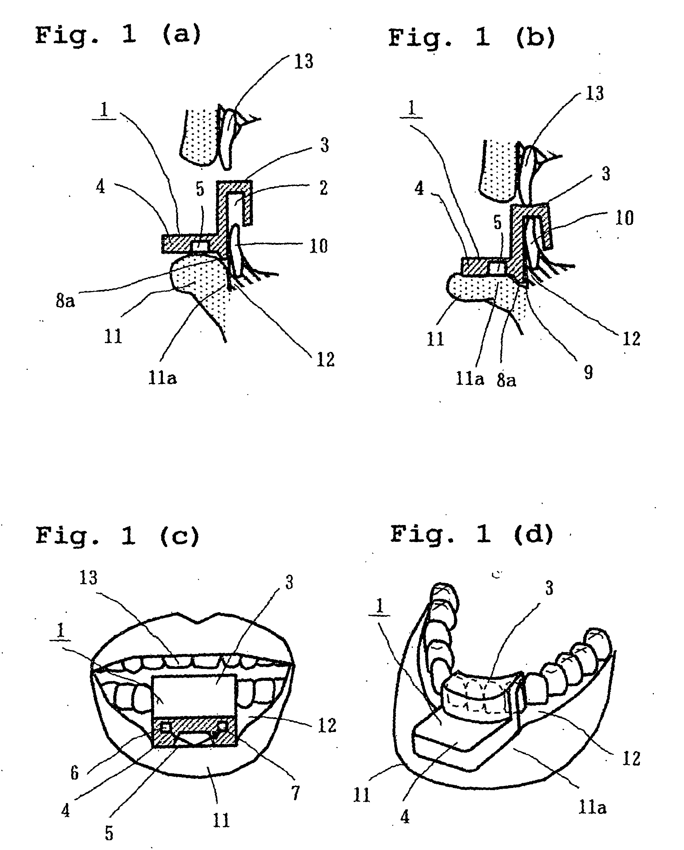

Fig. 1 shows:
   (a) a cross sectional view of imagery regarding how a vital information measuring device according to a preferred embodiment 1 of the present invention is used;
   (b) a cross sectional view of imagery regarding how the vital information measuring device according to the preferred embodiment 1 of the present invention is used;
   (c) a front view of imagery regarding how the vital information measuring device according to the preferred embodiment 1 of the present invention is used; and
   (d) a schematic view of imagery regarding how the vital information measuring device according to the preferred embodiment 1 of the present invention is used;

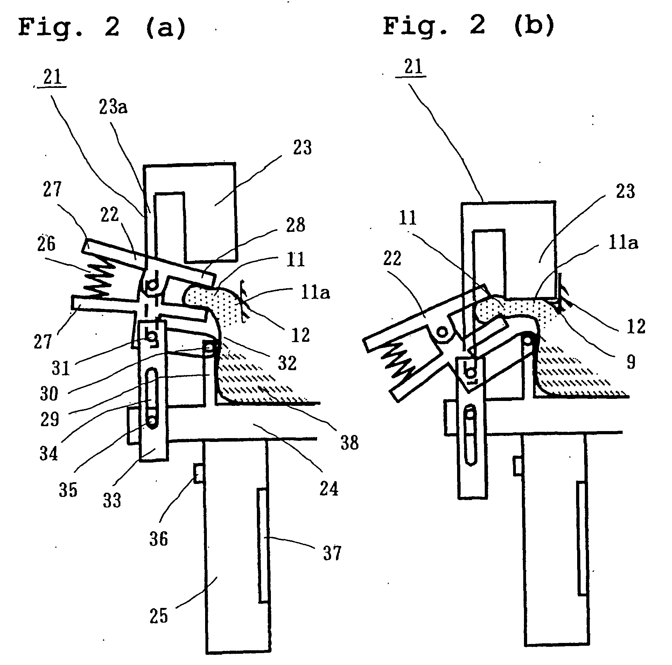

Fig. 2 shows:
   (a) a cross sectional view of imagery regarding how a vital information measuring device according to a preferred embodiment 2 of the present invention is used; and
   (b) a cross sectional view of imagery regarding how the vital information measuring device according to the preferred embodiment 2 of the present invention is used;
Fig. 3 shows:
   (a) a partial cross sectional view as it is looked at from the front which shows the vital information measuring devices according to the preferred embodiments 1 and 2 of the present invention; and
   (b) an enlarged view of the same;
Fig. 4 is a partial appearance view of the vital information measuring device according to the preferred embodiment 2 of the present invention;
Fig. 5 shows:
   (a) a cross sectional view of imagery regarding how a vital information measuring device according to other preferred embodiment of the present invention is used; and
   (b) a cross sectional view of imagery regarding how the vital information measuring device according to the other preferred embodiment of the present invention is used;

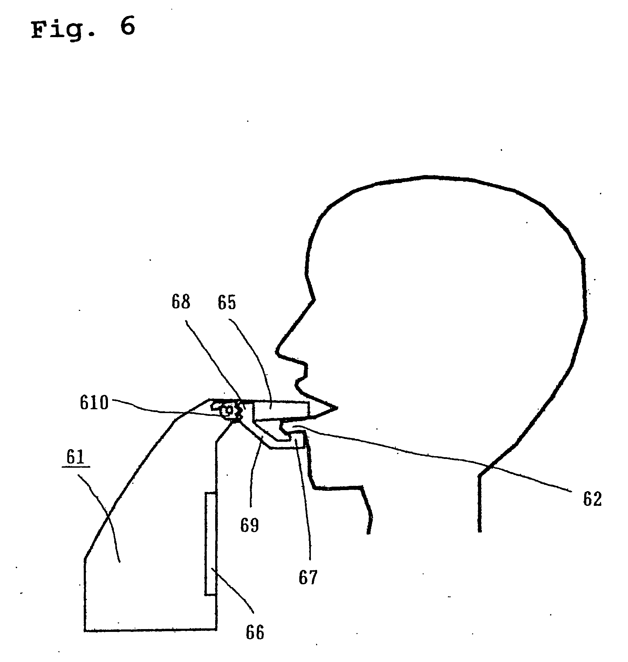

Fig. 6 is a cross sectional view of imagery regarding how a vital information measuring device according to a preferred embodiment 3 of the present invention is used;

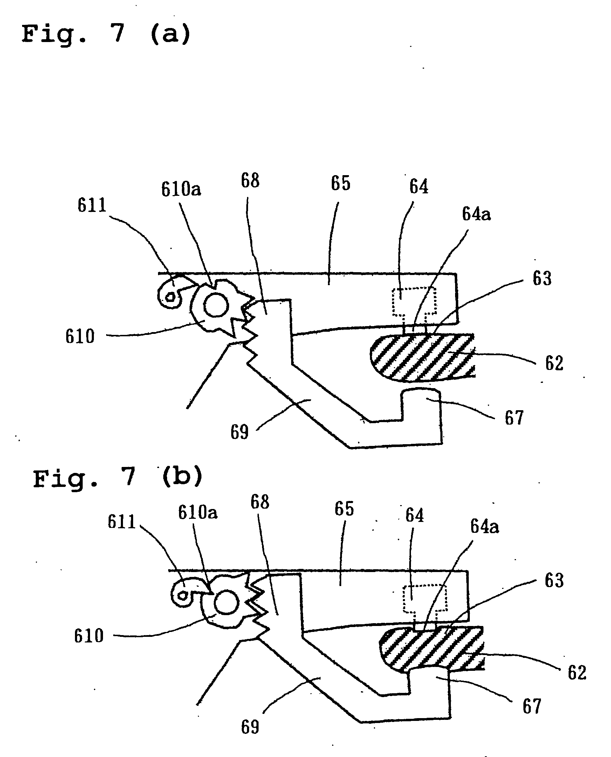

Fig. 7 shows:
   (a) a partially detailed view of the vital information measuring device according to the preferred embodiment 3 of the present invention; and
   (b) a partially detailed view of the vital information measuring device according to the preferred embodiment 3 of the present invention;

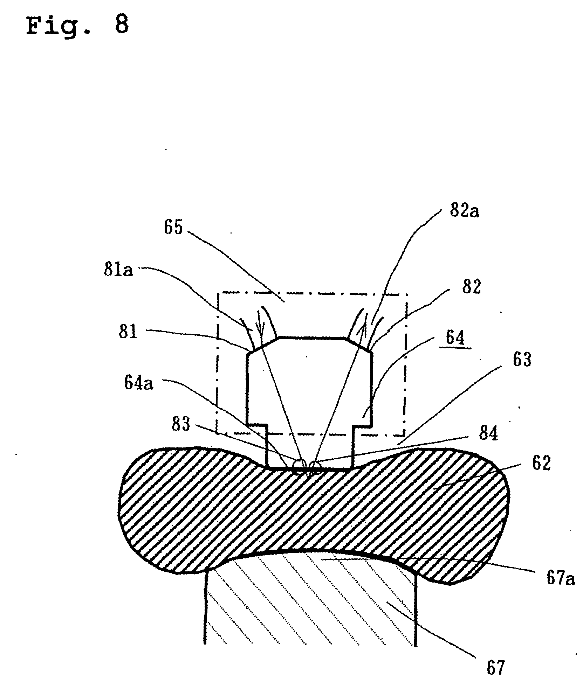

Fig. 8 partially enlarges the vital information measuring device according to the preferred embodiment 3 of the present invention;

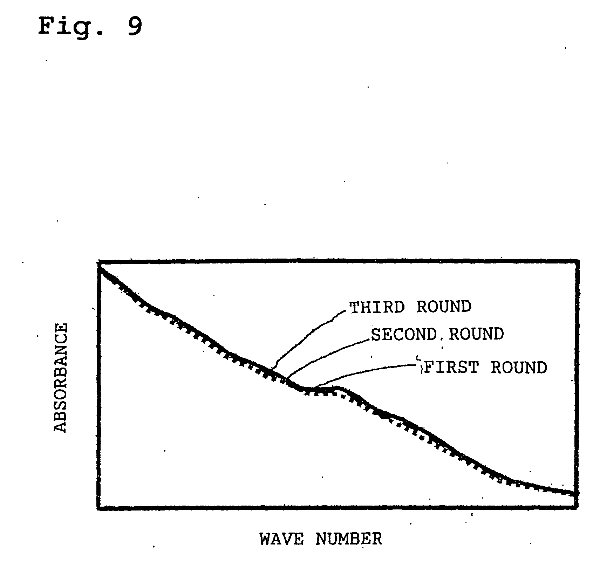

Fig. 9 partially enlarges spectra measured on a lip of a living body;

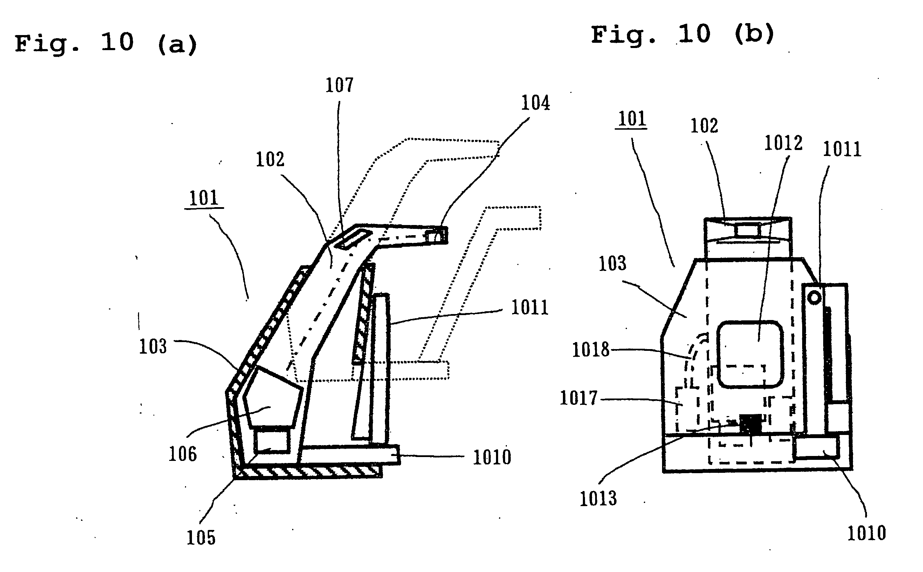

Fig. 10 shows:
   (a) a side cross-sectional view of a vital information measuring device according to a preferred embodiment 4 of the present invention; and
   (b) a front view of the vital information measuring device according to the preferred embodiment 4 of the present invention;

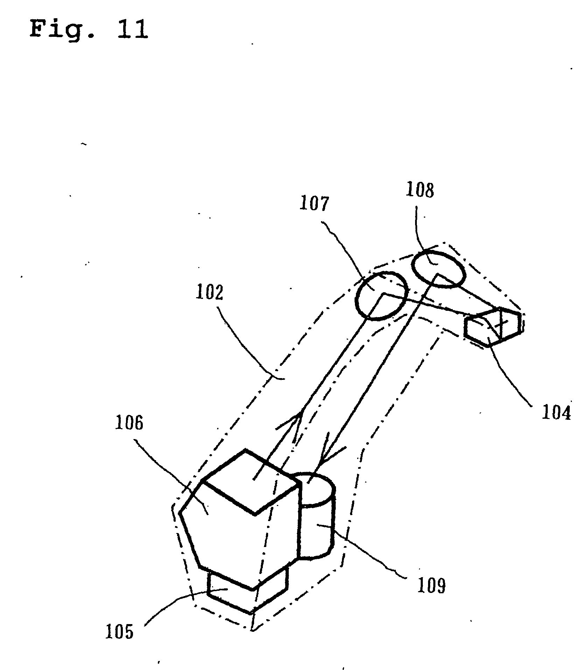

Fig. 11 is a partially detailed view of the vital information measuring device according to the preferred embodiment 4 of the present invention;

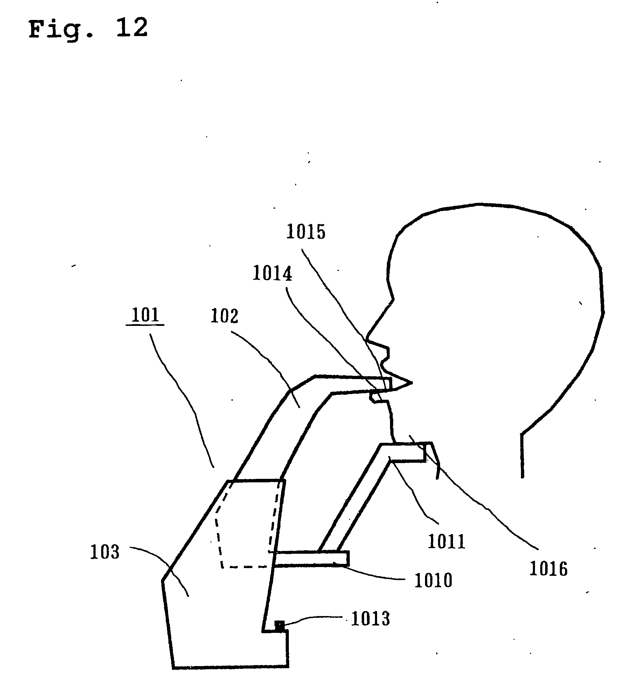

Fig. 12 is a drawing which shows imagery regarding how the vital information measuring device according to the preferred embodiment 4 of the present invention is used;
Fig. 13 is a schematic view which shows one example of a vital information measuring device according to a conventional example;
Fig. 14 is a graph of spectra measured in relation to the conventional example;
Fig. 15 shows:
   (a) a drawing of spectra measured on water in relation to the conventional example;
   (b) a drawing of the state of water corresponding to the spectra according to the conventional example;
   (c) a drawing of the state of water corresponding to the spectra according to the conventional example; and
   (d) a drawing of the state of water corresponding to the spectra according to the conventional example;
Fig. 16 partially enlarges spectra measured on water in relation to the conventional example; and
Fig. 17 partially enlarges spectra measured on a lip of a living body in relation to the conventional example.

### (EXPLANATION OF THE REFERENCE SYMBOLS)

- 1: mouthpiece-type vital information measuring device
- 3: fitting part
- 4: measuring part
- 9: frenulum labii inferioris
- 10: lower incisor teeth
- 11: lower lip
- 11a: labial mucosa
- 12: gum
- 61: tabletop vital information measuring device
- 62: lip
- 64: prism
- 65: measuring part
- 67: lip supporting part
- 69: auxiliary arm
- 610: positioning lever
- 611: stopper
- 101: tabletop vital information measuring device
- 102: measuring part
- 104: prism
- 105: light source
- 106: interferometer
- 107: light guiding mirror
- 108: detection mirror
- 109: detector

### BEST MODE FOR IMPLEMENTING THE INVENTION

With reference to the associated drawings, embodiments of the present invention will now be described.

### (Embodiment 1)

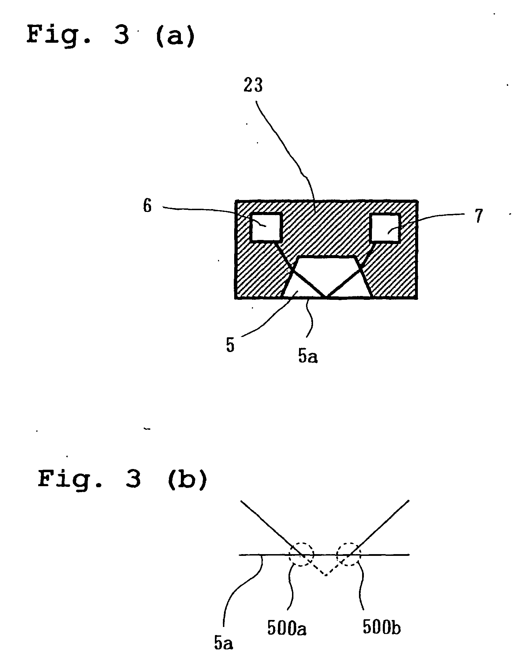

Figs. 1(a), (b) are cross sectional views of imagery regarding how a vital information measuring device according to an embodiment 1 of the present invention is used, Fig. 1(c) is a front view of imagery regarding use, Fig. 1(d) is a schematic view of imagery regarding use, Fig. 3 (a) is a partial cross sectional view as it is looked at from the front, and Fig. 3(b) is an enlarged view.

In Figs. 1(a) through (d), denoted at 1 is a mouthpiece-type vital information measuring device which comprises a fitting part 3 having a positioning concave portion 2 which fits along lower incisor teeth 10 and a measuring part 4 which contacts on a lower lip 11. The measuring part 4 incorporates a prism 5 in such a manner that a contacting surface 5a of the prism contacts on the lower lip 11, and light emitted from a light emitting element 6 moves forward within the prism 5 as denoted at the arrow and effuses to the lower lip 11, which is a subject-under-measurement, via an outgoing area 500a which is on the contacting surface 5a. Transmitted light, reflected light, scattered light or transmitted and reflected light after thus effused further moves forward within the prism 5 via an incident area 500b which is on the contacting surface 5a, and is detected by a light receiving element 7. Light to be measured may include all or some of transmitted light, reflected light, scattered light or transmitted and reflected light.

Electric driving means for the measuring part 4, such as transmission to the light emitting element 6 and transmission from the light receiving element 7, will not be shown in drawings. A fiber may be used as an information detecting contact shoe. Further, to the side of the measuring part 4 on which the lower lip 11 contacts, a press-open projection 8a for pressing open the lower lip 11 is disposed.

Operations of the mouthpiece-type vital information measuring device 1 according to this embodiment having such a structure as described above are as follows. The positioning concave portion 2 disposed to the fitting part 3 of said mouthpiece-type vital information measuring device 1 is fit to the lower incisor teeth 10. At this stage, the fitting is performed such that the press-open projection 8a will always be located between the lower lip 11 and the lower gum 12 while lightly pressing the press-open projection 8a resting on the gum 12 against the lower incisor teeth 10.

In this condition, owing to the elasticity of the lower lip 11, the positioning concave portion 2 of the mouthpiece-type vital information measuring device 1 is still off and above the lower incisor teeth 10.

The positioning concave portion 2 disposed to the fitting part 3 contacts on the lower incisor teeth 10 as the fitting part 3 is pressed by upper incisor teeth 13 in this state, the press-open projection 8a moves down to a frenulum labii inferioris 9 (tendons linking the lower lip and the gum) and accordingly presses open the lower lip 11, thereby deforming the lower lip 11 such that a' labial mucosa 11a which is usually in contact with the gum 12 will be exposed to outside the mouth. Since the fitting part 3 is held as it is pressed and held by the upper incisor teeth 13 at this stage, the measuring part 4 closely adheres to the lower lip 11.

In short, the prism 5 incorporated within the measuring part 4 closely adheres the labial mucosa 11a of the lower lip 11 without fail.

When the upper and the lower teeth mesh with each other, the position of the prism 5 is restricted from moving on the labial mucosa 11a in the mouthpiece shape, and hence, as long as a user is the same person, a measurement position on the labial mucosa which is a portion-under-measurement is easily and securely reproduced.

The position reproducibility is made even more secure by such a structure in which a contact sensor is disposed to said press-open projection 8a resting on the gum 12 and whether the labial mucosa 11a resting on the gum 12 is correctly exposed is confirmed based on contact with the frenulum labii inferioris 9.

The contact confirmation using such a contact sensor may be replaced with confirmation of an image using an ultra-compact CCD camera or the like.

Further, while this embodiment uses the structure that the mouthpiece shape is fit to the lower incisor teeth 10, a mouthpiece shape fitting all of the lower teeth may be used. In short, anything which uses the row of the teeth as the positioning means, presses the lip open, exposes the labial mucosa and make the measuring part 4 adhere will do.

### (Embodiment 2)

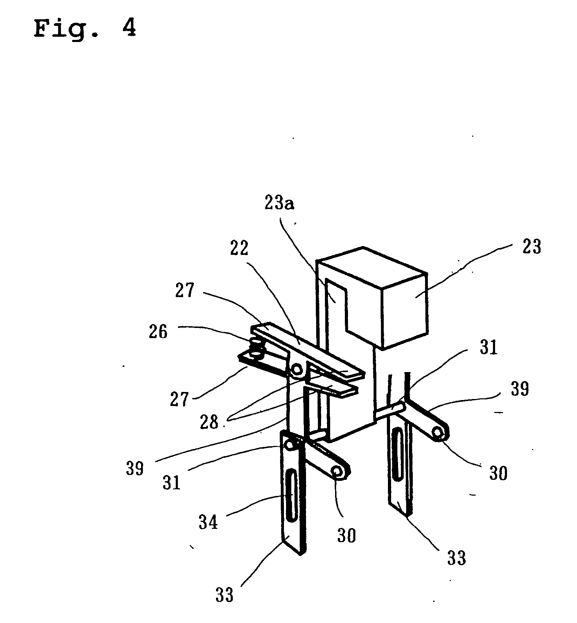

Figs. 2 (a) , (b) are cross sectional views of imagery regarding how a vital information measuring device according to an embodiment 2 of the present invention is used. Fig. 3 is a partial cross sectional view as it is looked at from the front, of which (a) is a partial cross sectional view as it is looked at from the front and (b) is an enlarged view, and Fig. 4 is a partial appearance view of the vital information measuring device according to the embodiment 2.

In Figs. 2(a), (b) and 4, denoted at 21 is a vital information measuring device which comprises a clip part 22 which pinches the lower lip 11, a measuring part 23 which contacts on the lower lip 11, a positioning stage 24 on which a jaw 38 is to be rested and a grip part 25 which one holds in his or her one hand.

The clip part 22 is comprised of an elastic member 26, hand/finger holding sections 27 and lip pinching sections 28. An L-shaped member 39 is attached below the clip part 22, a front end portion of the L-shaped member 39 is mounted for free revolutions to a reference pin 30 of a reference axis 29 which is disposed to the positioning stage 24, and a moving pin 31 is fixed to a bent portion of the L-shaped member 39.

The reference pin 30 is located in the vicinity of or below a sulcus mentolabialis 32. Further, a slide plate 33 is hung from the moving pin 31 for free rotations. Still further, a slide slit 34 formed at the center of the slide plate 33 is engaged with a guiding pin 35 of said positioning stage 24.

In addition, there is another clip part 22 comprising the L-shaped member 39 and another slide plate 33 disposed beyond the measuring part 23 (Fig. 4 shows a part of them.).

Said moving pin 31 links the respective L-shaped members 39 as shown in Fig. 4. Further, a holding part 23a is fixed to the moving pin 31, and said measuring part 23 is held at the top end of this holding part 23a.

The prism 5 comprising the contacting surface 5a, the light emitting element 6 and the light receiving element 7 are incorporated within the measuring part 23 as shown in Fig. 3 (a). As in the embodiment 1, light from the light emitting element 6 moves forward within the prism 5 as denoted at the arrow and effuses to the lower lip 11, which is a subj ect-under-measurement, via the outgoing area 500a which is on the contacting surface 5a. Transmitted light, reflected light, scattered light or transmitted and reflected light after thus effused further moves forward within the prism 5 via the incident area 500b which is on the contacting surface 5a, and is detected by the light receiving element 7. Light to be measured may include all or some of transmitted light, reflected light, scattered light or transmitted and reflected light.

Electric driving means for the measuring part 4, such as transmission to the light emitting element 6 and transmission from the light receiving element 7, will not be shown in drawings. A fiber may be used as an information detecting contact shoe.

Operations of the vital information detecting device 21 according to this embodiment having such a structure as described above are as follows.

As one holds the hand/finger holding sections 27 of the clip part 22, accordingly opens the lip pinching sections 28 and releases the hand/finger holding sections 27 while holding the lower lip 11, as shown in Fig. 2(a), the lower lip 11 becomes pinched by the clip part 27. At this stage, the measuring part 23 is not in contact with the lower lip 11.

Next, as one moves the slide plates 33 along the guiding pin 35 toward below, the clip part 22 fixed to said slide plates 33 'rotates about the reference pin 30 of the positioning stage 24 and moves down. As this occurs, the lower lip 11 pinched by the clip part 22 is deformed as if it were pulled together with the clip part 22 toward below, thereby exposing the labial mucosa 11a, which is usually in contact with the gum 12, to outside the mouth.

At the same time with this, the measuring part 23 linked to the clip part 27 by the L-shaped members 39 moves down in accordance with the rotation of the clip part 27. This makes the contacting surface 5a of the prism 5 closely adhere to and contact on the labial mucosa 11a of the lower lip 11 without fail.

The method of moving said slide plates 33 may be manual, or alternatively, automatic utilizing a combination with a motor, etc. A stopper not shown in the drawings fixedly holds the slide plates 33 in this state, and after measurement has ended, the slide plates are released from the stopper automatically or manually.

According to this embodiment, since the positioning stage 24 and the reference axis 29 thus restrict the position of the jaw, the positions of the measuring part 23, the clip part 22 and the lower lip 11 always remain unchanged as long as a user is the same person, which ensures that a measurement position on the lower lip 11 at which the measuring part 23 takes measurement is easily and securely reproduced.

In the embodiments described above, the prism 5 corresponds to the first contacting means of the present invention, the contacting surface 5a corresponds to the contacting surface of the present invention, the outgoing area 500a corresponds to the outgoing area of the present invention, and the incident area 500b corresponds to the incident area of the present invention. The fitting part 3 and the measuring part 4 or the measuring part 23, the positioning stage 24, the slide plates 33, the moving pin 31, the reference pin 30 and the guiding pin 35 correspond to the fixing means of the present invention, and the clip part 22 corresponds to the pinching means of the present invention, the labial mucosa 11a corresponds to the portion-under-measurement of the present invention. Further, the press-open projection 8a corresponds to the press-open means of the present invention.

While the foregoing has described that the light emitting element 6 and the light receiving element 7 are incorporated within the measuring part 4 of the mouthpiece-type vital information measuring device 1, the light emitting element 6 and the light receiving element 7 may be disposed outside so that light will be transmitted through an optical fiber or the like. In this case, the mouthpiece-type vital information measuring device 1 corresponds to the vital information detecting contact device of the present invention, and the vital information detecting contact device may be structured to be disposable, which is hygienic.

Although not shown in the drawings, if there is pressing means which presses the measuring part 23 from the bottom surface of the lower lip 11 as if to pinch the measuring part 23, the degree of close adhesion further improves at the stage that the measuring part 23 adheres.

In addition, if a pressure sensor is disposed to this pressing means, it will be possible to confirm the pressure of the measuring part 23 which closely adheres to the lower lip 11, and hence, automatically achieve close adhesion under a constant pressure.

While the structure of the clip part 22 is such a structure which pinches at two locations on the both sides while leaving positions at the center of the lip open according to the embodiments described above, an alternative structure may be used in which the clip part 22 pinches the lower lip 11 at only one location in the vicinity of the center of the lower lip. Further alternatively, a structure which pinches at three or more locations may be used. In short, any structure may be used which exposes the labial mucosa by pinching and pulling the lip or cheek.

In addition, a measurement-purpose driving circuit, a data memory circuit and the like are incorporated within the grip part 25 although not shown in the drawings, a switch 36 and a display panel 37 for starting measurement, driving the stopper and other purposes are disposed in the surface of the grip part 25, and measurement data are accordingly displayed.

The measurement-purpose driving circuit, the data memory circuit and the display panel however may be disposed outside instead of incorporating these within the grip part 25, so that the grip part,25 will be used simply as a tool for gripping. In this case, the vital information measuring device 21 corresponds to the vital information detecting contact device of the present invention.

Further, although the measuring part 23 is moved linearly from above toward below in the embodiment 2, an alternative structure may be used in which the measuring part rotates like a fan from an oblique direction: In short, the only requirement is that after the lower lip 11 is pulled, the measuring part 23 contacts on and is pressed against the labial mucosa 11a.

Further, other than restriction of the jaw, positioning may be attained utilizing restriction of the head like one provided by a headphone or restriction of the row of teeth as in the embodiment 1: The point is that a position on a portion-under-measurement of the same person is easily and securely reproduced and adhesion to the portion-under-measurement is secured.

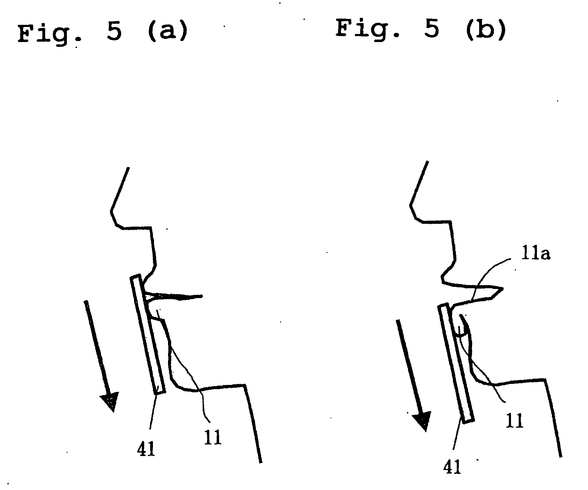

Further, Figs. 5 (a) , (b) show other embodiment of the present invention, wherein without pinching a lip, a thin plate 41 for example is pressed as a contacting member against the front surface of the lower lip 11, which is protruded somewhat beyond an upper lip, in such a manner that the thin plate will not slip off from the lip, and the thin plate is then moved along the jaw direction as denoted at the arrow in this state, whereby the lower lip 11 closely adhering to the thin plate 41 is pulled similarly in the direction of the jaw and the labial mucosa 11a is exposed off from the gum.

Further, while the lower lip is a portion-under-measurement in the embodiments, there will be no problem if the labial mucosa of the upper lip is measured as a portion-under-measurement. In this case, the device may be handled upside down in both the embodiments 1 and 2, so that the deforming means of the present invention would deform the upper lip and expose the upper lip to outside. The gist of the present invention lies in that a tissue such as a labial mucosa which rests on gum and is usually hidden is exposed without fail and measurement is carried out with the contacting means contacting on and tightly adhering to the tissue and that the measurement position is securely reproduced.

### (Embodiment 3)

Fig. 6 is a drawing of imagery regarding how a vital information measuring device according to an embodiment 3 of the present invention is used, Figs. 7(a), (b) are partially detailed views of this vital information measuring device, and Fig. 9 shows an example of spectra measured on a lip of a living body.

In Figs. 6 and 7 (a) , (b) , denoted at 61 is a tabletop vital information measuring device which internally comprises a measuring part 5 whose front end has a prism 64 which contacts on a labial mucosa 63 of a lower lip 62, and although not shown in the drawings, a light source, light guiding means which makes light from this light source impinge upon the prism 64 and further move forward within the prism 64 after effusing to a labial mucosa 3 which is a contacting subject-under-measurement, and a detector which receives this light.

Further, Fig. 8 is a partially enlarged front view which shows a section in the vicinity of the prism 64. Formed in the prism 64 are an incident end 81 upon which light guided from the light source by the light guiding means impinges, an outgoing end 82 from which transmitted light, reflected light, scattered light or transmitted and reflected light as it is after effused to the labial mucosa 63 leaves and a contacting surface 64a which directly contacts the labial mucosa 3, optical fibers 81a and 82a are connected respectively with the incident end 81 and the outgoing end 82, the other end of the optical fiber 81a is connected with the light source which is not shown in the drawings, and the other end of the optical fiber 82a is connected with the detector which is not shown in the drawings.

The light guiding means may not be optical fibers, and light may of course be guided by an optical system which uses a plurality of mirror surfaces.

The contacting surface 64a of the prism 64 which contacts on and senses out the labial mucosa 63 which is a subject-under-measurement has a structure that the sensing area size is a small size which is equal to or smaller than half the lip surface area and protrudes beyond the surface of said measuring part 65, and includes an outgoing area 83 from which light entering at the incident end 81 and moving through the prism 64 leaves toward the labial mucosa 3 and an incident area 84 upon which transmitted light, reflected light, scattered light or transmitted and reflected light impinges which develops as light coming from the outgoing area 83 effuses to the labial mucosa. Light to be measured may include all or some of transmitted light, reflected light, scattered light or transmitted and reflected light.

A calculation circuit not shown in the drawings is incorporated inside the tabletop vital information measuring device 61, thereby forming a structure that data from said measuring part 65 are accepted and processed and a display part 66 disposed to a front or rear surface displays the same.

An auxiliary arm 69, which comprises at one end a lip supporting part 67 supporting a lip from below and at the other end a gear rack 68 which is for movements, is attached for sliding to the measuring part 65. Further attached to the measuring part 65 is a positioning lever 610 having a gear surface which meshes with the gear rack 68 of the auxiliary arm 69, and to the positioning lever 610, lever fixing means formed by a stopper 611 and a notch 610a is disposed.

The positioning lever 610 may be manual one which is revolved with a hand for instance, or alternatively, automatic one which operates using a known technique such as a motor and a gear mechanism: Any structure may be used which achieves positioning and fixing by means of the stopper 611 and the notch 610a after sliding. Further alternatively, the lever fixing means may use other known technique than the stopper 611 and the notch 610a.

A structure that a measurement start button becomes operable upon fixing of the positioning lever 610 may be used, or a structure that fixing of the positioning lever 610 is detected and measurement automatically starts may be used.

A distance which the auxiliary arm 69 travels as the positioning lever revolves is susceptive to individual differences depending upon a person who serves as a subject-under-measurement and becomes different among different subjects-under-measurement, and therefore, is defined as follows. First, the thickness of a lip of a person who will be involved in measurement is measured in advance. Following this, a gap between the contacting portion 64a of the prism 64 and the lip supporting part 67 is determined to be narrower than thus identified thickness. For instance, the gap is set to be thinner by 2 mm than the measured thickness of the lip. The stopper 611 is disposed at thus set position, thereby preventing the positioning lever 610 from further rotating.

The lip supporting part 67 is as large as or larger than the contacting portion 64a, and is shaped as a slightly round convex surface. In this convex surface, a height difference between a central portion and an end portion is 0.5 mm or smaller.

Operations of the tabletop vital information measuring device 61 according to the embodiment 3 having such a structure as above will now be described, which will also give a description on an embodiment of a vital information measuring method according to the present invention.

With the lip 62 inserted between the measuring part 65 and the auxiliary arm 69 and with the labial mucosa 63 accordingly brought into contact with the prism 64, the positioning lever 610 is rotated, whereby the lip supporting part 67 contacts on the lip 62. As the positioning lever 610 is further revolved until the positioning lever 610 gets restricted by the stopper 611, the lip supporting part 67 moves, the lip 62 is pressed against the prism, and the labial mucosa 63 closely adheres to the contacting portion 64a which is a sensing surface in consequence. At this stage, since the gap between the lip supporting part 67 and the contacting portion 64a of the prism 4 is set to be narrower by 2 mm than the thickness of the lip, the lip gets pressed and pressurized and owing to its own elasticity closely adheres to the contacting portion 64a. In addition, since the surface area size of the contacting portion 64a is smaller than the lip surface, the force upon the pressing surface gets concentrated, thereby increasing the degree of adhesion.

Since the lip supporting part 67 is as large as or larger than the contacting portion 64a, the labial mucosa 63 which has contacted the contacting portion 64a is pressed against the entire contacting portion 64a without fail. Further, the pressurization eliminates the minute unevenness of the lip 62 itself, which in turn suppresses the interposition of an air layer.

Further, as shown particularly in Fig. 8 in expansion, since the size of the pressurized adhesion area is small and has the slightly round part 67a, saliva on the labial mucosa 3 easily escapes from the center of the contacting portion 64a to the periphery which is not pressurized by the contacting portion 64a and the lip supporting part 67, and variations of a layer of saliva on the labial mucosa portion 6 under adhesion are extremely small.

While the distance which the auxiliary arm 69 travels is set to be less than the thickness of the lip, the dimensions may be within a range of 0.5 mm to 5 mm, and an appropriate amount may be initially set in accordance with the elasticity of the lip of the subject-under-measurement, the thickness of the lip, etc.

Further, the position of the positioning lever at a lip thickness position may be temporarily stored as a zero position every time measurement is taken, and the position may then be automatically slid 2 mm for instance after thus set to zero.

Further, while the lip supporting part 67 is movable in this embodiment, the prism 64 may be moved and pressed against: In short, the lip supporting part 67 or the prism 64 moves, the lip supporting part 67 and the contacting portion 64a of the prism 64 are accordingly pressed against the lip 62 until they intrude into the lip, and the labial mucosa 63 and the contacting portion 64a closely adhere to each other. At this stage, the lip supporting part 67 may not intrude into the lip 62. The pressure may be onto such an extent that at least the contacting portion 64a intrude into the lip 62.

As an operation switch of the device 61 is turned on, light intrudes the labial mucosa 63 from the outgoing area 83 of the contacting portion 64a via the light splitting means and the prism 64 from the internally disposed light source, transmitted light, reflected light, scattered light or transmitted and reflected light impinges upon the incident area 84 after effusing to the labial mucosa 63 and is then received by the detector via the outgoing end 82, and the display part 66 of the device 61 displays blood sugar value data obtained by the calculation circuit, as described above. The data are saved'in a memory circuit although not shown in the drawings, and therefore, past data can be retrieved at any desired time.

Since the stopper 611 fixedly holds the positioning lever 610 and measurement is taken only in the fixed state, no measurement is taken when pressing is halfway through and adhesion is instable, thereby attaining a stable pressing reproducibility.

Further, using a simple structure that the distance which the auxiliary arm 69 comprising the lip supporting part 67 travels is set but fixed after moving, a one-touch action realizes close adhesion to the labial mucosa 63 in a simple manner without fail.

Although the vital information measuring device is of a tabletop type in this embodiment, the device may be of a portable type. Alternatively, a pressure sensor corresponding to the pressure measurement means of the present invention may be incorporated inside the lip supporting part 67 to thereby read the pressure force at the time of pressing. This eliminates the necessity of measuring the thickness of the lip of the subject-under-measurement in advance, but allows to quantitatively judge a distance between the lip supporting part 67 and the prism 64 and to appropriately press just to such an extent that adhesion indents the labial mucosa 63 of the subject-under-measurement.

Further, a power mechanism which operates based on the pressure measured by the pressure sensor may be disposed, to thereby appropriately control the amount of rotation of the positioning lever 610.

Fig. 9 partially enlarges spectra on a lip obtained through measurement in this embodiment in which measurement was taken successively three times with the lip and the prism in close adhesion in a similar fashion to that according to the conventional example shown in Fig. 7. Throughout the first to the third round of measurement, spectra having approximately equal profiles were obtained.

In other words, when measurement is taken according to the present invention, variations are extremely small and a measurement reproducibility is high.

In the embodiment described above, the prism 64 corresponds to the first contacting means of the present invention, the contacting surface 64a corresponds to the contacting surface of the present invention, the outgoing area 83 corresponds to the outgoing area of the present invention, and the incident area 84 corresponds to the incident area of the present invention. The lip supporting part 67 corresponds to the second contacting means of the present invention, while the auxiliary arm 69, the positioning lever 610 and the stopper 611 correspond to the distance fixing means of the present invention. Meanwhile, the labial mucosa 63 corresponds to the portion-under-measurement of the subject-under-measurement of the present invention. The calculation circuit corresponds to the calculating means of the present invention, and the vital information detecting contact device according to the present invention corresponds to what is formed by the prism 64, the lip supporting part 67, the stopper 611 and the positioning lever 610.

While the foregoing has described that the lip supporting part 67 has the slightly round part 67a, the purpose of this is to protect lips against injury: When other portion is to be measured, the lip supporting part may have a pointed convex part instead of a round part.

Further, although the foregoing has described that the tabletop vital information measuring device 61 may be integrated with the prism 64, the lip supporting part 67, the stopper 611 and the positioning lever 610, since implementation requires only the prism 64, the lip supporting part 67, the stopper 611 and the positioning lever 610, the vital information detecting contact device of the present invention may have a structure which is separate from a main unit section which contains the calculation circuit, the light source and the like. This is hygienic, as the structure of the vital information detecting contact device is disposable.

In addition, while the foregoing has described that blood sugar value data are to be measured, the particular component referred to in the present invention may be data representing fats, proteins or the like other than this. Alternatively, in addition to a lip, the above may be used for measurement of other portions, such as the inside of a mouth, which secrets a mucosa. These are similarly applicable to the embodiments 1 and 2.

### (Embodiment 4)

Fig. 10(a) is side cross-sectional view of a vital information measuring device according to an embodiment 4 of the present invention, Fig. 10(b) is a front view of this vital information measuring device, Fig. 11 is a partially detailed view of this vital information measuring device, and Fig. 12 is a drawing which shows imagery regarding use.

In Figs. 10 through 13, denoted at 101 is a tabletop vital information measuring device in which a measuring part 2, whose front end mounts a prism 104 contacting on a labial mucosa 1015 of a lower lip 1014, is incorporated inside a holder stage 3. The structures of details of the prism 104 are similar to those according to the embodiments 1 through 3, and therefore, will not be described again.

Disposed inside the measuring part 102 are a light source 105, an interferometer 106 to which light from the light source 105 is guided and which then splits the light, a light guiding mirror 107, a detection mirror 108 and a detector 109.

Although not shown in the drawings, the inside of the interferometer 106 is formed by a beam splitter, a fixed mirror and a movable mirror. Light impinges upon the beam splitter and is split into two luminous fluxes which will then be reflected respectively by the fixed mirror and the movable mirror and returned back to the beam splitter, at which stage the luminous fluxes when in the same phase intensify with each other but weaken each other when in different phases, thus giving rise to a interference phenomenon, which then ensures that light having different intensities owing to the phase difference attributed to an optical path difference is guided to the prism 104 by the light guiding mirror 107, further moves forward within the prism 104 after effusing into the labial mucosa 1015 which is the contacting subject-under-measurement, and is then received by the detector 109 because of the detection mirror 108.

In other words, since all of the light is directly transmitted and received via the mirrors, a transmission loss which will be created during transmission through an optical fiber, does not occur, thereby obtaining a stable signal. While the interferometer 6 is one example of the light splitting means of the present invention, other examples are a grading-type spectrometer which splits light by means of diffraction and an interferometer filter-type spectrometer which limits a particular wavelength.

Further, in this fashion, in the measuring part 102, the holding member of the present invention is formed and the optical system formed by the light source 105, the interferometer 106, the light guiding mirror 107, the prism 104, the detection mirror 108 and the detector 109 is held as one unit inside this, and hence, the optical axis, the optical path length and the like are constant and a stable signal is obtained.

A movement lever 1010 which serves as moving means is fixed to the measuring part 102 so that the measuring part 102 itself will be able to slide, and the movement lever 1010 protrudes for free sliding beyond said holder stage 3.

A folding-type jaw stage 1011 which serves as the position restricting member of the present invention is fixed to an end portion of the movement lever 1010, thereby realizing a structure which can adjust the height although not shown in the drawings. With the position restricted by the jaw stage 1011, as long as a user is the same person, a measurement position on the labial mucosa 1015 which is a portion-under-measurement is securely reproduced in a simple manner without fail.

The measure which slide-moves may be manual means which is lifted up with a hand, hooked to a stopper, a notch or the like at the lever and fixed for instance, or automatic means which is operated using a known technique such as a motor and a gear mechanism.

Further, a calculation circuit 1017 is incorporated inside the holder stage 103, thereby forming a structure that data from the measuring part 102 are received via a circuit cable 18 or the like, processed, and displayed by a display 1012 which is disposed to the front surface.

The calculation circuit 1017 may be disposed inside the measuring part 102.

Operations of the tabletop vital information measuring device 101 according to this embodiment having such a structure as described above are as follows.

When the movement lever 1010 is lifted up, the measuring part 102 moves obliquely toward above and is fixed at that position by a stopper not shown in the drawings. The folded jaw stage 1011 is then opened and fixed. With a jaw 1016 resting on the jaw stage 1011, the prism 104 is made touch the labial mucosa 1015 of the lower lip 1014 in such a manner that the contacting surface of the prism closely adheres to the labial mucosa, and as an operation switch 1013 of the holder stage 103 is pressed, light intrudes the labial mucosa 1015 from the light source 105 inside the measuring part 102 via the interferometer 106 and the prism 104 and is then received by the detector 109, and the display 1012 of the holder stage 103 displays blood sugar value data which are obtained by the calculation circuit 1017, as described above.

The data are saved in a memory circuit although not shown in the drawings, and therefore, past data can be retrieved at any desired time.

In this manner, the measuring part 102 is made as a moving structure such that the measuring part will be placed at such a position and made to take such a posture which allow easy measurement only when measurement is to be taken, which in turn realizes a compact device which will be used as a tabletop device yet will not be a hindrance when put on a table or desk and which therefore could always be left on a table as if the device were an interior fixture. Therefore, whenever one has noticed not only after a meal, he or she can easily take measurement. In addition, since painful blood collection is not necessary, one can casually take measurement as many times as he or she wishes during one day.

Further, although the measuring part 102 moves obliquely toward above in the structure according to this embodiment, the measuring part may move along the upright direction: In short, a person who is to take contacting-type measurement on the lower lip 1014 is allowed to take an easy posture.

Further, although the measuring part 102 is formed as a moving structure according to this embodiment, the measuring part may be fixed on the holder stage 3: In short, using an optical system in which neither the optical path length nor the optical axis changes but the both remain constant, a stable signal accompanying no transmission loss is obtained.

Further, the structure of the tabletop vital information measuring device according to this embodiment may be realized in combination with the vital information measuring devices according to the embodiments 1 through 3.

Although the foregoing has described that the living body serving as the subject-under-measurement in the present invention is a human body and the portion-under-measurement is a labial mucosa in each embodiment, in the present invention, any other portion on which at least the first contacting means needs to contact, such as the inner wall inside the mouth, gum, lids and genital organs, may be used. Alternatively, any desired position on a human body may be used. In addition, the living body referred to in the present invention is not limited to a human being but may be other animal, and a tissue of other animal may be the portion-under-measurement referred to in the present invention.

### POSSIBILITY OF INDUSTRIAL USE

As described above, the present invention permits to reproduce a position on the portion-under-measurement in a simple manner and accordingly attains highly precise measurement.

## Claims

1. A vital information detecting contact device, comprising:
first contacting means having an contacting surface which contacts on a portion-under-measurement of a subject-under-measurement; and
fixing means which fixes such that said contacting surface will not move on said portion-under-measurement,
wherein said contacting surface comprises an outgoing area, from which light leaves to said portion-under-measurement, and an incident area upon which all or some of transmitted light, reflected light, scattered light or transmitted and reflected light based on light thus leaving said outgoing area from said portion-under-measurement impinges upon.

2. The vital information detecting contact device of claim 1, comprising deforming means which deforms at least a part of said subject-under-measurement such that said portion-under-measurement will be able to contact on said contacting surface.

3. The vital information detecting contact device of claim 2, wherein said subject-under-measurement is a lip portion of a living body, said portion-under-measurement is a labial mucosa of said lip portion, and
said deforming means deforms said lip portion such that said labial mucosa will be exposed to outside.

4. The vital information detecting contact device of claim 3, wherein said deforming means comprises press-open means which is held inside the mouth of said living body and heads toward a frenulum labii inferioris from gum outside teeth.

5. The vital information detecting contact device of claim 2, comprising a fitting part which meshes with upper and lower teeth inside the mouth of said living body,
wherein said first contacting means is disposed outside the row of teeth, and as said fitting part meshes with said upper and lower teeth, said contacting surface closely adheres to said labial mucosa.

6. The vital information detecting contact device of claim 3, wherein said deforming means comprises firm holding means which pinches said lip portion at one place at least.

7. The vital information detecting contact device of claim 6, wherein said firm holding means is structured so as to revolve about a sulcus mentolabialis or a portion below the sulcus mentolabialis.

8. The vital information detecting contact device of claim 7, wherein said fixing means comprises holding means which holds said first contacting means, moves when said firm holding means revolves in association with the revolution of said firm holding means, and makes said contacting surface contact on said lip portion.

9. The vital information detecting contact device of claim 1, wherein said subject-under-measurement is a living body, and
said fixing means realizes said fixing when supported by at least one of the row of teeth, the jaw and the head of said living body.

10. The vital information detecting contact device of claim 1, further comprising second contacting means which is located facing said first contacting means beyond said portion-under-measurement, and contacts on a different portion from said portion-under-measurement of said subject-under-measurement, and
said fixing means fixes so that said first contacting means and said second contacting means will be maintained at a constant distance with each other.

11. The vital information detecting contact device of claim 10, wherein said distance fixing means is capable of setting said constant distance to a plurality of distances which are different from each other.

12. The vital information detecting contact device of claim 10, wherein the surface area size of said contacting surface is smaller than the surface area size of a portion on which said second contacting means contacts.

13. The vital information detecting contact device of claim 10, wherein said second contacting means has a substantially convex shape.

14. The vital information detecting contact device of claim 13, wherein said convex shape has a round part.

15. The vital information detecting contact device of claim 10, comprising pressure measurement means which measures the pressure upon said portion-under-measurement applied by said first contacting means and/or said second contacting means.

16. The vital information detecting contact device of claim 15, wherein said pressure measurement means changes said constant distance based on said measured pressure.

17. A vital information measuring device, comprising:
the vital information detecting contact device of any one of claims 1 through 16;
a light source which sends light to said outgoing area of said contacting surface;
light receiving means which receives light from said incident area of said contacting surface; and
calculating means which calculates a light receipt result of said light receiving means and acquires information regarding said portion-under-measurement.

18. The vital information measuring device of claim 17, further comprising confirmation means which confirms a place deformed by said deforming means.

19. The vital information measuring device of claim 18, wherein said confirmation means is contact confirmation by means of a contact sensor.

20. The vital information measuring device of claim 18, wherein said contact confirmation utilizes an end portion of said press-open means.
